# EUROPEAN PATENT APPLICATION

(11) **EP 4 406 477 A1**
(43) Date of publication of application: **31.07.2024**
(21) Application number: 24153222.5
(22) Date of filing: 22.01.2024
(51) Int. Cl.: A61B 5/0245, A61B 5/0537, A61B 5/06, A61B 5/282, G16H 50/20, A61B 5/256, A61B 5/029, A61B 5/0507, A61B 5/08, A61B 8/00

(54) **VISCERAL BODY COMPOSITION MEASUREMENT ARRANGEMENT**

(30) Priority: 24.01.2023 GB 202300993
(71) Applicant: Polar Electro Oy, 90440 Kempele (FI)
(72) Inventor: KORKALA, Seppo, 90440 Kempele (FI)
(74) Representative: Kolster Oy Ab

(57) **Abstract**

This document discloses a wearable body composition measurement system, comprising: a sensor device comprising a first measurement circuitry configured to measure body composition, a second measurement circuitry configured to measure heart activity, and a strap arranged to attach the sensor device around a torso of a user; at least one processor coupled with the sensor device; and at least one memory storing a computer program code configured to cause the at least one processor to perform operations comprising: in a first measurement mode where the sensor device is attached around an abdominal area of the user, receiving body composition measurement data measured by the first measurement circuitry, computing a quantity of fat tissue in the abdominal area on the basis of the received body composition measurement data, and outputting the quantity of fat tissue via an interface; and in a second measurement mode where the sensor device is attached around a chest of the user, receiving heart activity measurement data from the second measurement circuitry, computing at least one heart activity parameter on the basis of the received heart activity measurement data, and outputting the at least one heart activity parameter via the interface.

## Description

### TECHNICAL FIELD

The invention relates to a field of body composition measurement sensors and, particularly, to a measurement arrangement for measuring body composition at abdominal area.

### TECHNICAL BACKGROUND

Visceral body fat also called hidden fat is fat stored deep inside the body, around internal organs such as the liver. It has been commonly acknowledged as related to serious medical diseases such as heart and arterial diseases and diabetes. However, known solutions for measuring the visceral fat call for better measurement arrangements. There exist various body composition measurement arrangements implemented in a scale or in a device gripped by the user by both hands. The problem with these is that they measure the body composition of whole lower body or upper body. Some devices then estimate the amount of visceral fat on the basis of total fat percentage and theoretical knowledge that visceral fat makes about ten percent of the total body fat. Some other solutions are based on measuring the waist circumference and mapping the waist circumference to the amount of visceral fat. While there exists correlation between these characteristics and the amount of visceral fat, such methods based on rules of thumb call for improvement.

### BRIEF DESCRIPTION

According to a first aspect, there is provided: a wearable body composition measurement system, comprising: a sensor device comprising a first measurement circuitry configured to measure body composition, a second measurement circuitry configured to measure heart activity, and a strap arranged to attach the sensor device around a torso of a user; at least one processor coupled with the sensor device; and at least one memory storing a computer program code configured to cause the at least one processor to perform operations comprising: in a first measurement mode where the sensor device is attached around an abdominal area of the user, receiving body composition measurement data measured by the first measurement circuitry, computing a quantity of fat tissue in the abdominal area on the basis of the received body composition measurement data, and outputting the quantity of fat tissue via an interface; and in a second measurement mode where the sensor device is attached around a chest of the user, receiving heart activity measurement data from the second measurement circuitry, computing at least one heart activity parameter on the basis of the received heart activity measurement data, and outputting the at least one heart activity parameter via the interface.

The system may further comprise a training computer. The training system may house the at least one processor and the at least one memory. The training system may comprise a wireless communication circuitry configured to couple the at least one processor with the sensor device that is external to the training computer. The training system may comprise a third measurement circuitry configured to measure a body composition, comprising a set of electrodes arranged to contact with limbs of the user. The at least one processor may be configured in the first measurement mode to control the third measurement circuitry to measure the body composition via the set of electrodes and to generate further body composition measurement data, to compute a further quantity of fat tissue on the basis of said further body composition measurement data, and to output the further quantity of fat tissue via the interface.

At least one processor may be configured to combine the body composition measurement data with the further body composition measurement data.

According to a further aspect, there is provided: a computer-implemented method comprising: connecting with a sensor device comprising a first measurement circuitry configured to measure body composition, a second measurement circuitry configured to measure heart activity, and a strap arranged to attach the sensor device around a torso of a user; in a first measurement mode where the sensor device is attached around an abdominal area of the user, receiving body composition measurement data measured by the first measurement circuitry, computing a quantity of fat tissue in the abdominal area on the basis of the received body composition measurement data, and outputting the quantity of fat tissue via an interface; and in a second measurement mode where the sensor device is attached around a chest of the user, receiving heart activity measurement data from the second measurement circuitry, computing at least one heart activity parameter on the basis of the received heart activity measurement data, and outputting the at least one heart activity parameter via the interface.

According to a further aspect there is provided: a computer program product embodiment on a computer-readable distribution medium storing computer program instructions that, when read and executed by a computer, cause the computer to carry out a computer process comprising: connecting with a sensor device comprising a first measurement circuitry configured to measure body composition, a second measurement circuitry configured to measure heart activity, and a strap arranged to attach the sensor device around a torso of a user; in a first measurement mode where the sensor device is attached around an abdominal area of the user, receiving body composition measurement data measured by the first measurement circuitry, computing a quantity of fat tissue in the abdominal area on the basis of the received body composition measurement data, and outputting the quantity of fat tissue via an interface; and in a second measurement mode where the sensor device is attached around a chest of the user, receiving heart activity measurement data from the second measurement circuitry, computing at least one heart activity parameter on the basis of the received heart activity measurement data, and outputting the at least one heart activity parameter via the interface.

The embodiments and features, if any, described in this specification that do not fall under the scope of the independent claim are to be interpreted as examples useful for understanding various embodiments of the invention.

### BRIEF DESCRIPTION OF THE DRAWINGS

In the following the invention will be described in greater detail by means of preferred embodiments with reference to the attached drawings, in which
Figure 1 illustrates a wearable measurement system according to an embodiment;
Figure 2 illustrates measurement arrangements and associated measurement procedure according to an embodiment;
Figure 3 illustrates arrangement of electrodes in a wrist-worn training computer and a procedure using them in body composition measurements according to an embodiment;
Figure 4 illustrates a procedure for controlling the utilization of measurement circuitries of a sensor device according to an embodiment;
Figure 5 illustrates a computer-implemented procedure for conducting body composition measurements according to an embodiment;
Figure 6 illustrates a procedure for automated detection of attachment location of a sensor device according to an embodiment;
Figure 7 illustrates a measurement arrangement and associated measurement procedure according to an embodiment;
Figure 8 illustrates a measurement arrangement and associated measurement procedure according to another embodiment;
Figure 9 illustrates an apparatus for a training computer according to an embodiment;
Figure 10 illustrates an apparatus for a sensor device according to an embodiment.

### DETAILED DESCRIPTION OF THE INVENTION

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

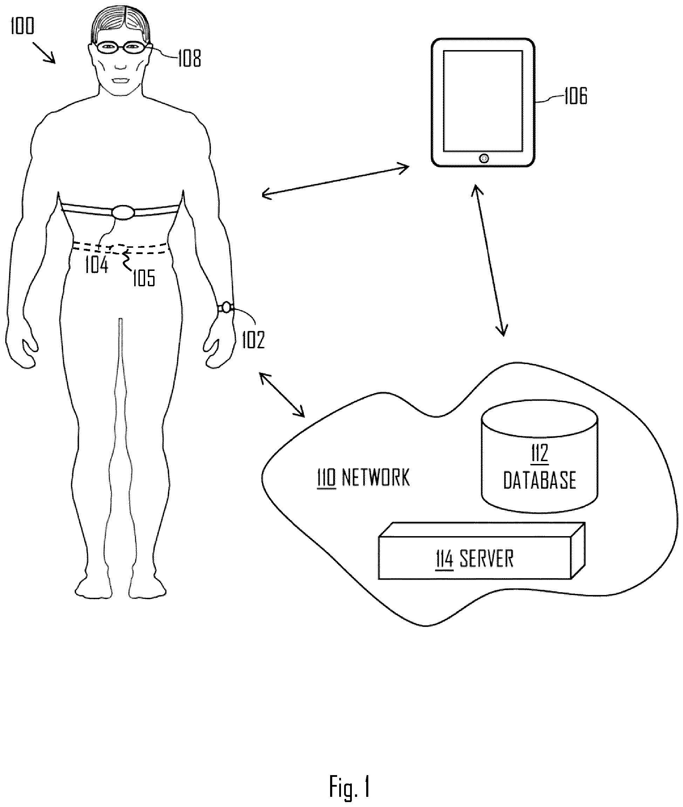

Figure 1 illustrates an example of a wearable sensor system to which the embodiments of the invention may be applied. Referring to Figure 1, a user 100 wearing one or more devices may be shown. The devices may comprise a wrist device 102, one or more external sensor devices 104, 105, and optionally goggles or eyewear 108 for user interfacing. The sensor devices 104, 105 can be understood as separate devices or as a single device having multiple attachment location and associated location-specific functions, as described below. Accordingly, the sensor device 105 attached around the abdominal area of the user may be understood as a sensor device additional to the sensor device 104 attached around the chest area or as the sensor device 104 attached to the abdominal area. The wrist device 102 is an example of a training computer configured to control measurements and also process measurement data collected by the external sensor device(s) and also sensor devices internal to the training computer. Further, the system of Figure 1 may comprise a portable electronic device 106 and/or network 110 (e.g. a training network) comprising at least one database 112 and at least one server 114. The server 114 may be a local server or it may be a cloud server accessible through the Internet.

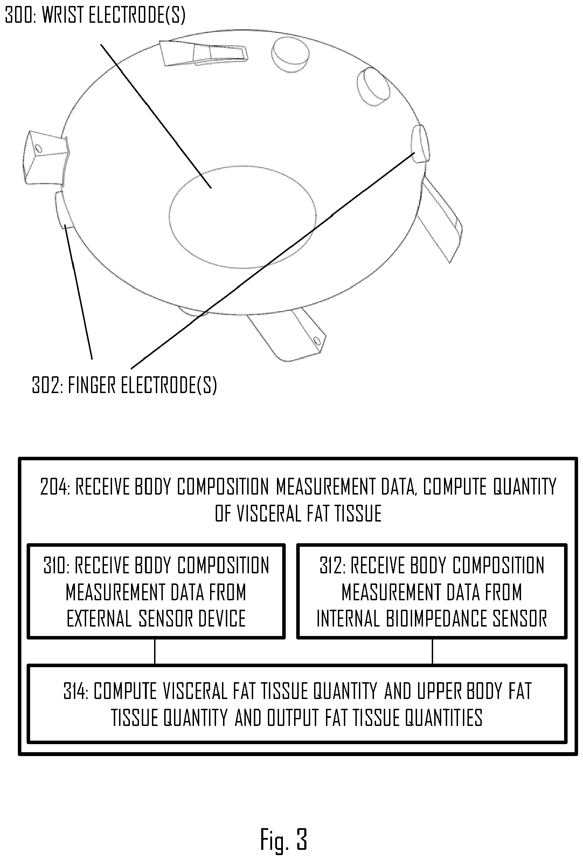

The wrist unit 102 may comprise one or more sensor configured to measure physical activity of the user 100. Examples of these sensors may comprise motion sensors such as accelerometer(s), gyroscope(s), satellite positioning circuitry (e.g. a global navigation satellite system (GNSS) circuitry, such as a Global Positioning System (GPS) and/or a GLObal NAvigation Satellite System (GLONASS). Further examples of these sensors include heart activity sensors that include an optical heart activity sensor configured to measure the heart activity via photoplethymogram (PPG) and an electric heart activity sensor configured to measure the heart activity via electrocardiogram (ECG). Yet further examples of these sensors include a blood oximetry (SpO2) sensor and a body composition sensor. The body composition sensor may be based on measurements of bioimpedance, as described in greater detail below with reference to Figure 3. The wrist unit 102 may be used to monitor physical activity of the user 100 by the user 100, for example. Thus, the wrist unit 102 may comprise a display and other conventional elements of a user interface. Further, the wrist device 102 may comprise communication circuity enabling wireless and/or wired data communication with other device(s) of the system. The eyewear may form a part of the user interface, e.g. for displaying at least some of the parameters computed by the training computer.

The one or more external sensor devices 104, 105 may comprise sensor device(s) worn by the user 100 and/or sensor device(s) which may be used by the user 100. For example, a heart activity belt 104, 105 may be worn by the user 100 as shown in Figure 1. The external sensor device(s) 104, 105 may comprise sensors, such as a heart activity sensor, a motion sensor, a positioning sensor, and a bioimpedance sensor, to mention a few. The heart activity sensor may be configured to measure electrocardiogram or impedance cardiogram. An example of the impedance cardiogram measurements is stroke volume measurement. In the embodiments described below, the external sensor device(s) 104, 105 is/are realized as chest sensor(s) configured to be placed around the user's 100 torso. For that purpose, the sensor device(s) 104, 105 may comprise one or more measurement circuitries configured to measure physiological characteristics of the user and, additionally, comprise a strap configured to attach the measurement circuitry around the torso. The external sensor device(s) 104 may measure the physiological characteristics and transmit associated measurement data to the wrist device 102, to some other wearable device, to the portable electronic device 106 and/or to a server 114, wherein the server is accessible via the network 110. Any one of these devices may function as a training computer configured to compute one or more physiological parameters on the basis of the measurement data and, in some embodiments, control the conduction of the measurements, as described in greater detail below. In some embodiments, the one or more physiological parameters may be computed in the external sensor device(s) 104, 105 and then transmitted to the training computer. The wrist device 102, the portable electronic device 106 and/or the server 114 may receive said sensor data. Thus, the external sensor devices(s) 104 may comprise wireless and/or wired communication capabilities. Further, the external sensor device(s) 104 may comprise one or more memories for storing data, such as the measurement data. During swimming, for example, it may be beneficial to load the measurement data to an external device after the swimming session is over due to possible data connection problems caused by water.

The portable electronic device 106, such as mobile phone, tablet computer, laptop or similar device, may also be used to monitor physical activity of the user 100. For example, in some implementations, the wrist unit 102 may not be necessary as the sensor device(s) 104 may transmit data directly to the server 114 and/or to the portable electronic device 106. Thus, the portable electronic device 106 may be used to view or monitor data concerning the physical activity (e.g. from memory of the portable electronic device 106) or via the network 110 (e.g. data stored in the database 112). Also in some implementations, the wrist unit 102 may transmit data to the portable electronic device 106 and/or the server 114.

The system described in relation to Figure 1 may be suitable for monitoring physical activity during different kinds of sports and also daily activities of the user. A conventional implementation of the chest sensor 104, 105 is a heart activity sensor configured to measure the user's electrocardiogram (ECG) non-invasively during a physical exercise and/or in a physiological test such as a fitness test. In such a case, a measurement circuitry of the chest sensor comprises an ECG sensor comprising, typically, a housing for the electronics of the ECG sensor and for the wireless communication circuitry and electrodes arranged in the strap. The electrodes may be elongated to ensure that they extend sufficiently around the torso and, as a consequence, the sensor device and measurement circuitry also extends around the user's torso.

With the development of sensor devices, further features have been incorporated into the chest sensors. For example, a motion sensor configured to measure the motion of the torso is included in some commercially available chest sensors. Patent publication US 2020/0060576 discloses in connection with Figures 22 and 23 a chest sensor configured to measure bioimpedance. As known in the art, the bioimpedance sensor may be used for non-invasive body composition measurements. Such a chest sensor may be used in the embodiments described below. It may also be used for measuring the heart activity (the stroke volume), as described in the pertinent patent publication. In the body composition measurements, an alternating current (AC) may be induced into the user's body via current electrodes of the chest sensor while voltage electrodes of the chest sensor measure voltage from the user's body. The AC may be induced at multiple frequencies for better resolution of the body composition. Such bioimpedance measurements for the purpose of measuring the body composition are in some literature called bioelectrical impedance analysis. As known in the art, different tissues have different electric characteristics that can be measured via such bioimpedance measurements. Fat tissue, muscular tissue and bone tissue all conduct electric current with unique characteristics.

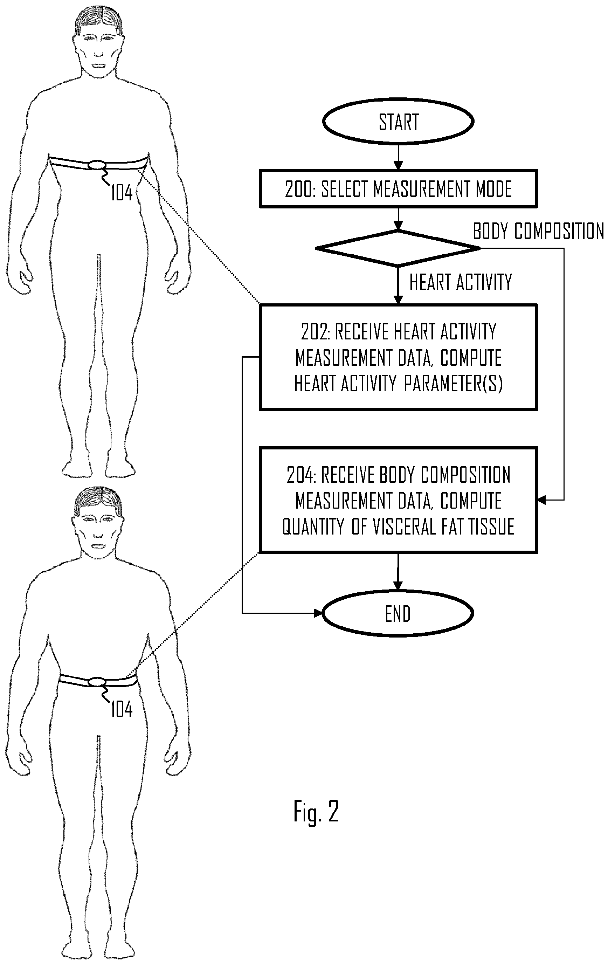

Thanks to the capability for body composition analysis, the chest sensor described above has hardware needed for measuring also the visceral fat. Below, embodiments using the chest sensor for measuring the visceral fat are described. Referring to Figure 2, the described embodiments relate to a wearable body composition measurement system, comprising: a sensor device 104 comprising a first measurement circuitry configured to measure body composition, a second measurement circuitry configured to measure heart activity, and a strap arranged to attach the sensor device around a torso of a user; at least one processor coupled with the sensor device; and at least one memory storing a computer program code configured to cause the at least one processor to perform operations comprising:
in a first measurement mode where the sensor device is attached around an abdominal area of the user, receiving (block 204) body composition measurement data measured by the first measurement circuitry, computing a quantity of fat tissue in the abdominal area on the basis of the received body composition measurement data, and outputting the quantity of fat tissue via an interface; and
in a second measurement mode where the sensor device is attached around a chest of the user, receiving (block 202) heart activity measurement data from the second measurement circuitry, computing at least one heart activity parameter on the basis of the received heart activity measurement data, and outputting the at least one heart activity parameter via the interface.

As described above, the measurement data may be processed by the at least one processor that selects a measurement mode (block 200) according to a determined criterion. Upon selecting the first measurement mode, a body composition measurement mode, block 204 may be carried out and the quantity of visceral fat tissue may be measured and computed. Upon selecting the second measurement mode, a heart activity measurement mode, block 202 may be carried out and the heart activity parameter(s) may be measured and computed.

From the perspective of a computer program executing the above-described method, the computer-implemented method may comprise: connecting with a sensor device comprising a first measurement circuitry configured to measure body composition, a second measurement circuitry configured to measure heart activity, and a strap arranged to attach the sensor device around a torso of a user; in a first measurement mode where the sensor device is attached around an abdominal area of the user, receiving body composition measurement data measured by the first measurement circuitry, computing a quantity of fat tissue in the abdominal area on the basis of the received body composition measurement data, and outputting the quantity of fat tissue via an interface; and in a second measurement mode where the sensor device is attached around a chest of the user, receiving heart activity measurement data from the second measurement circuitry, computing at least one heart activity parameter on the basis of the received heart activity measurement data, and outputting the at least one heart activity parameter via the interface. The connection may be wired or wireless, depending on whether or not the computer (processor(s)) is in the same casing with the measurement circuitries.

An advantage is more accurate measurement of the visceral fat, thanks to the measurements being directed specifically to the abdominal area by the dimensions of the strap and the arrangement of the measurement circuitry in the strap.

As described above, the bioimpedance sensor US 2020/0060576 may be comprised in the first measurement circuitry. However, another embodiment may utilize another type of body composition sensor. For example, the following sensors have been used as body composition sensors and are applicable for measuring the visceral fat in the context of the present invention: a radio frequency scanner, an ultrasound scanner, and an optical scanner. There exists scientific literature of using ultrasound as a reliable and precise method for measuring the amount of visceral fat and distinguishing the visceral fat from the subcutaneous fat. Similarly, an impulse radar based on ultra-wide band (UWB) technology has been introduced as a body composition analyser. The impulse radar is an example of a radio frequency scanner. A three-dimensional optical scanner is another possible technology.

In an embodiment, the second measurement circuitry is configured to measure at least one of a heart rate and stroke volume of the user, e.g. during a physical exercise. As described in US 2020/0060576, the stroke volume may be measured via bioimpedance. As a consequence, the second measurement circuitry may use at least partially the same electric components as the first measurement circuitry, e.g. at least the electrodes. The difference between the stroke volume measurements and the body composition measurements may be based on the differences between impedance cardiography and bioimpedance body composition analysis acknowledged in the literature. For example, the used frequencies may differ such that the stroke volume measurements may be carried out on a single frequency while the body composition measurements are multifrequency measurements. Stroke volume has been taught to be measured by using a frequency of about 30 to 50 kilohertz (kHz), while body composition measurements may be carried out in multiple frequencies selected from a wider frequency range, e.g. from 1 kHz or 5 kHz to 100 kHz or 200kHz or 250 kHz. Accordingly, the signal source generating the input current to the current electrodes may be different or at least configured differently in the first and second measurement circuitry.

In an embodiment, the body composition measurement focused on the abdominal area is used as a part of more holistic body composition measurements. As described above, the wrist device 102 may comprise a body composition measurement circuitry. Figure 3 illustrates an arrangement of bioimpedance measurement electrodes in the wrist device. For example, a first set of one or more electrode 300 may be arranged to contact the skin of the hand where the wrist device is worn while a second set of one or more electrode 302 may be arranged to be touched by the other hand to enable upper body bioimpedance measurements and body composition analysis. The first set 300 may be arranged on a bottom surface of a casing of the wrist device such that the first set contacts the skin of the wrist area of the hand where the wrist device is worn. The second set 302 may be arranged on a side surface or side surfaces of the casing and/or on a top surface of the casing such that the user may touch them with a finger or fingers during the measurements. Some prior art solutions have described electrodes even in a strap or band of the wrist device, and that is a possible implementation of the first and/or second set. as described above, at least one of the electrodes (preferably at least two) may be the current feed electrode while at least one of the electrodes (preferable at least two) may be the voltage measurement electrode. In case of two-plus-two electrodes, there may be arranged a current feed electrode and a voltage measurement electrode per hand.

As described above, the wrist device is an embodiment of the training computer. A similar bioimpedance measurement arrangement may be realized in the portable electronic device 106 as well, by providing electrodes the user 100 touches with both hands. The training computer may then house the at least one processor and the at least one memory, and the training computer may further comprise a wireless communication circuitry configured to couple the at least one processor with the external sensor device 104 that is external to the training computer. The training computer may have the bioimpedance measurement circuitry as a third measurement circuitry configured to measure the body composition, comprising the described set of electrodes arranged to contact with limbs of the user. The third measurement circuitry may further comprise other state-of-the-art electronics and software needed for measuring the bioimpedance and body composition in the wrist device.

In the embodiment of Figure 3, the at least one processor is configured in the first measurement mode to control the third measurement circuitry to measure (block 312) the body composition via the set(s) of electrodes 300, 302 and to generate further body composition measurement data, to compute (block 314) a further quantity of fat tissue on the basis of said further body composition measurement data, and to output the further quantity of fat tissue via the interface. Additionally, a quantity of one or more other type of tissue (bone, muscle) may be measured and computed in blocks 312, 314. Accordingly, the at least one processor may compute and output the quantity visceral fat tissue that is measured by the sensor device placed around the abdominal area (received in block 310) and the quantity of fat tissue measured and computed by using the body composition sensor of the wrist device. Accordingly, two quantities of fat tissue are acquired, and they may be output as separate parameters, e.g. displayed via the user interface.

In an embodiment of Figure 3, the at least one processor combines the body composition measurement data received in block 310 with the further body composition measurement data acquired in block 312. Since the body composition measurement data received from the external sensor is specifically focused measuring the abdominal area while the body composition measurement data acquired from the internal sensor represents the upper body composition, it may be beneficial to sum the quantities of the fat tissue or reduce at least some of the quantity of fat tissue in the abdominal area from the quantity of fat tissue in the upper body. Accordingly, the outputted combined quantity of fat tissue may better represent the quantity of fat tissue in the whole upper body area (hands, chest, abdominal area). In case there is overlapping between the measurement coverage of the body composition measurements, the summing or reduction may be weighted in some manner. The degree of summing or reduction can be discovered by experimentation and testing, and it may be specific to characteristics of the sensor device 104, 105, e.g. the arrangement of electrodes of the sensor device 104, 105 or scanning area of the sensor device 104, 105. In an embodiment, the (two) different quantities of fat tissue measured by the different measurement arrangements are output as separate quantities: one measured from the wrist labelled to indicate `upper body fat quantity' and the other measured by using the sensor device 104, 105 labelled to indicate 'abdominal area fat quantity'.

In yet another embodiment, the combining is understood as evaluating the body composition measurement data of block 310 and the body composition measurement data of block 312 in separate processing branches but then evaluating their trends or events thereof jointly. For example, the development of quantities of fat tissue may be monitored separately and the development of the quantity of upper body fat tissue with the development of the quantity of visceral fat tissue. There is some natural changes in the quantities because of daily/weekly variations in the user's habits to calorie consumption and calorie intake and because of measurement errors. However, if the development of one of the quantities changes from the development of the other one of the quantities, it may trigger an action and output of a notification to the user. For example, if the quantity of visceral fat tissue rises while the quantity of upper body tissue does not show similar development, it may cause output the notification of increased visceral fat tissue. On the other hand, if the quantity of visceral fat tissue changes (e.g. rises) and the quantity of upper body fat tissue experiences the same or similar change, this development may not cause the output of the notification. The notification may be user instruction to take actions to reduce the visceral fat, as described herein. It is possible that the quantity of muscle tissue has reduced, leading to increase in the relative quantity of fat tissue, for example. Accordingly, the increase of the fat tissue alone may not be a sufficient indicator for instructing the user to change habits.

The quantities of the fat and other tissues described herein may be absolute quantities or relative quantities. The relative quantity refers to relation with the quantities of other tissues (e.g. muscle and bone).

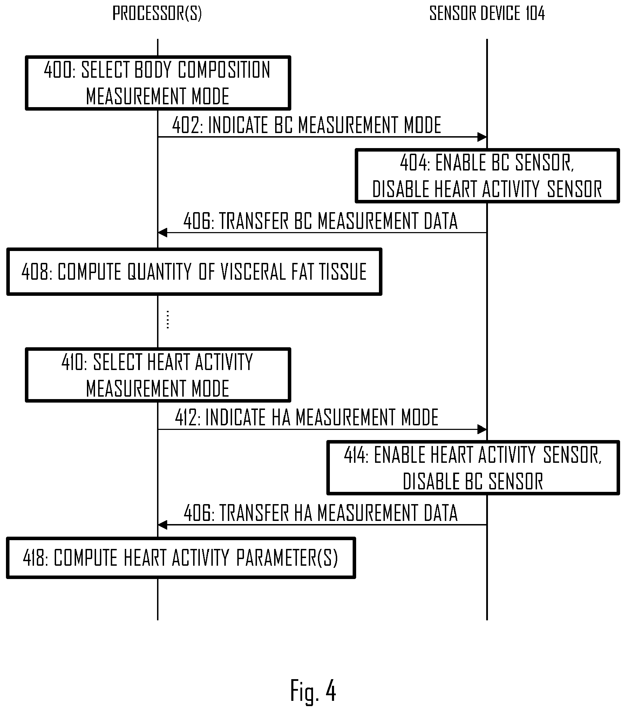

In an embodiment, the first measurement circuitry (body composition) is enabled and the second measurement circuitry (heart activity) is disabled in the first measurement mode (body composition measurement mode); and the second measurement circuitry is enabled and the first measurement circuitry is disabled in the second measurement mode (heart activity measurement mode). In this manner, power savings can be achieved. The enablement and disablement may be controlled by the at least one processor. In a standby mode, both (all) measurement circuitries may be disabled. Selection of either measurement mode may be triggered by user input. The heart activity measurement mode may be triggered when the user selects an exercise (e.g. a running exercise, swimming exercise, or cycling exercise) by operating the training computer. The selection of the exercise may cause the at least one processor to enable the heart activity measurement circuitry of the sensor device and keep the body composition measurement circuitry disabled. During the exercise, the heart activity measurement circuitry may thus measure and generate the heart activity measurement data such as heart rate measurement data and/or stroke volume measurement data. Alternatively, the heart activity measurement mode may be triggered by the user input selecting a fitness test or another test supported by the measurement system. An example of such another test is an orthostatic test. Yet alternatively, the heart activity measurement mode may be triggered upon detecting that the user has placed the sensor device 104, 105 into skin contact. State-of-the-art chest sensors have such a functionality to trigger the measurements and/or establish a wireless communication connection with the training computer. The body composition measurement mode may also be triggered by the user input, e.g. the user selecting a body composition measurement application by operating the training computer. The selection of the body composition measurement application may cause the at least one processor to enable the body composition measurement circuitry of the sensor device and keep the heart activity measurement circuitry disabled. Figure 4 illustrates a signalling diagram of the processor(s) enabling and disabling the measurement circuitries.

Referring to Figure 4, the processor(s) select(s) the body composition measurement mode in block 400, e.g. in response to the user input received via the user interface of the training computer. Upon selecting the body composition measurement mode, the processor(s) may send a signal to the sensor device over the wireless connection, for example, the signal indicating the body composition measurement mode (step 402). Upon receiving the signal, the sensor device may enable the body composition measurement circuitry (body composition sensor) and disable or keep disabled the heart activity sensor (block 404). In response to the enablement, the body composition measurement circuitry may start conducting the body composition measurements and generate the body composition measurement data. The sensor device 104 may then transmit the body composition (BC) measurement data wirelessly to the training computer (step 406) and the processor(s) may then use the body composition measurement data to compute the quantity of visceral fat tissue (block 408). The quantity of visceral fat tissue may be computed after all the measurement data has been transferred to the training computer. Accordingly, the body composition measurements can be understood as snapshot measurements.

Similarly, the processor(s) select(s) the heart activity measurement mode in block 410, e.g. in response to the user input received via the user interface of the training computer. Upon selecting the heart activity measurement mode, the processor(s) may send a signal to the sensor device over the wireless connection, for example, the signal indicating the heart activity measurement mode (step 412). Upon receiving the signal, the sensor device may enable the heart activity measurement circuitry (heart activity sensor) and disable or keep disabled the body composition sensor (block 414). In response to the enablement, the heart activity measurement circuitry may start conducting the heart activity measurements and generate the heart activity measurement data. The sensor device 104 may then transmit the heart activity (HA) measurement data wirelessly to the training computer (step 416) and the processor(s) may then use the heart activity measurement data to compute the heart activity parameter(s) (block 418). The computation may be continuous in the sense that new values of the heart activity parameters such as the heart rate and/or stroke volume may be computed as new heart activity measurement is received from the sensor device 104.

Both heart activity parameter(s) and the quantities of (visceral) fat may be used to generate user instructions. For example, the heart activity parameter(s) may be used to control the exercise according to an exercise plan, as known in the art. The quantities of fat may be used to guide the user in terms of exercising, nutrition, and sleep. For example, if the quantity of visceral fat tissue exceeds a threshold, the at least one processor may output an instruction to increase physical exercise and/or decrease nutrition, e.g. reduce food intake, reduce alcohol, and/or reduce fat or carbohydrate intake.

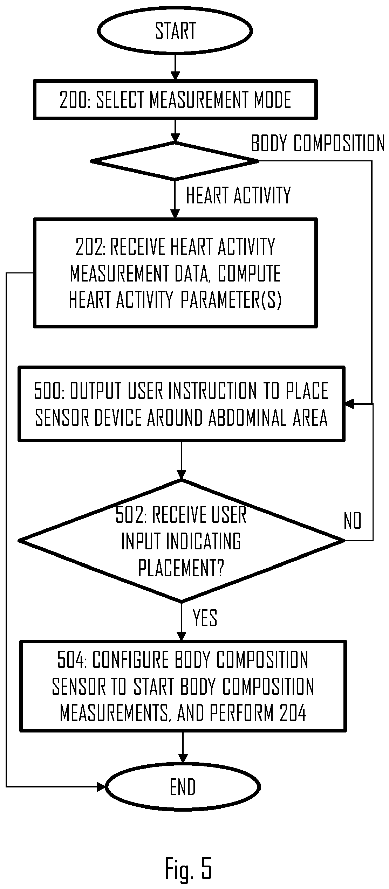

In an embodiment, the at least one processor is configured to, upon triggering the first measurement mode, to output an instruction to the user to place the sensor device around the abdominal area and, in response to a user input indicating that the sensor device has been placed around the abdominal area, configure the first measurement circuitry to start measuring the body composition measurement data. Figure 5 illustrates a flow diagram of such an embodiment.

Referring to Figure 5, the measurement mode may be selected in block 200 according to any one of the above-described embodiments. If the measurement mode is the heart activity measurement mode, the process may proceed to block 202, as described above. If the measurement mode is the body composition measurement mode, the process may proceed to block 500 where the at least one processor outputs through the user interface an instruction for the user to place the sensor device around the abdominal area. The instruction may be displayed or played via a loudspeaker. Then, the processor(s) may proceed to block 502 to wait for that the user acknowledges the placement of the sensor device. The instruction may be output repeatedly or continuously until the user acknowledgment is received via the user interface. Upon receiving the user acknowledgment, the process may proceed to block 504 where the processor(s) configure the body composition measurement circuitry of the sensor device to start the body composition measurements. Thereafter, the process may proceed in the above-described manner by the generation of the body composition measurement data and the computation and output of the quantity of the visceral fat tissue.

With respect to the heart activity measurements, such a user instruction about the placement of the sensor device may not be necessary. For example, the ECG may be measured from virtually any location of the user's body, including any location around the torso. Therefore, controlling the exact placement of the sensor device may not be as crucial in the heart activity measurement mode.

In an embodiment, the first (body composition) measurement circuitry is enabled in the first measurement mode and in the second measurement mode. For example, if the same measurement circuitry configuration is used for both the stroke volume and body composition measurements, the measurement circuitry may be enabled in both heart activity and body composition measurement modes. Alternatively, the configuration may be designed in a simplified form where the sensor device has all measurement circuitries enabled always when it is powered on. In such an embodiment, the at least one processor may be configured to, upon triggering the first measurement mode, to output an instruction to the user to place the sensor device around the abdominal area and, in response to a user input indicating that the sensor device has been placed around the abdominal area, start collection of the body composition measurement data for said computing the quantity of fat tissue in the abdominal area. Since the sensor device 104, 105 may be transmitting the body composition measurement data even before the user acknowledgment, the at least one processor may be configured to exclude from said computing at least some of body composition measurement data received before the user input. Accordingly, the measurement data collected before the sensor device was placed in the abdominal area cannot corrupt the computation of the quantity of fat tissue.

In an embodiment, the sensor device 104, 105 is configured to measure and detect the area of the torso to which the sensor device 104, 105 is currently attached. The body composition measurement circuitry may be used to measure the attachment location. As known from the human physiology, the tissues around the chest are different from the tissues around the abdominal area, e.g. there is more bone tissue around the chest. Therefore, a measurable fingerprint is also different in the chest area and in the abdominal area, which enables the detection of the attachment location. The fingerprint may be defined in terms of any one of the above-described measurable characteristics the body composition measurement circuitry is configured to measure, e.g. a bioimpedance fingerprint, a radio frequency fingerprint, an ultrasound fingerprint, or an optical fingerprint. The fingerprint may be recorded in a calibration phase when the sensor device 104, 105 is taken into use, or the fingerprint may have been predefined in terms of general knowledge of the different characteristics of the abdominal area and the chest area. In case the calibration phase is used, the training computer may control the calibration by instructing the user to place sensor device around the chest and, thereafter, configure the body composition measurement circuitry to record a fingerprint of the chest area, and by instructing the user to place sensor device around the abdominal area and, thereafter, configure the body composition measurement circuitry to record a fingerprint of the abdominal area. The fingerprints may then be stored in the at least one memory in a database 610. Accordingly, the database 610 stores first information on a measurement signal fingerprint of a situation where the sensor device is attached around the chest and second information on a measurement signal fingerprint of a situation where the chest first measurement circuitry is attached around the abdominal area. The first and second information may be stored in connection with the respective area of attachment. The database 610 may be stored in a memory of the sensor device 104, 105.

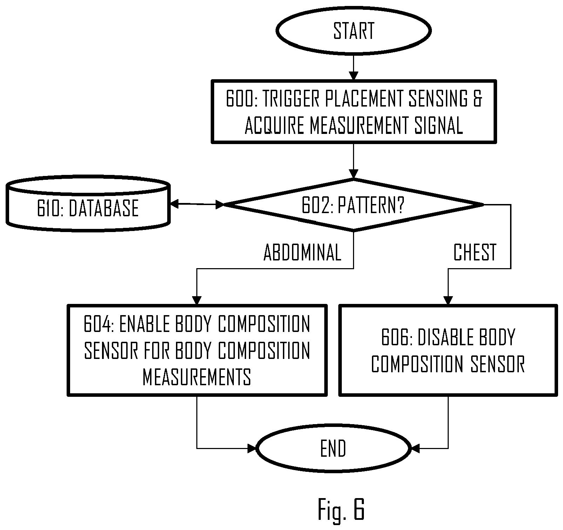

The sensor device 104, 105 may further comprise a detection circuitry configured to carry out the procedure of Figure 6. Referring to Figure 6, the detection circuitry may be configured to detect skin contact of the sensor device by using an electric or optical sensor, for example. Upon detecting the skin contact, the detection circuitry may trigger detection of the placement of the sensor device (block 600). Block 500 may comprise enabling the first measurement circuitry, the second measurement circuitry, or another measurement circuitry of the sensor device 104, 105 to start measurements. Upon receiving the measurement data from the enabled measurement circuitry, the detection circuitry may cross-reference the measurement data with the fingerprints stored in the database 610. The detection circuitry may select the fingerprint providing the greatest correlation with the measurement data and determine the attachment location associated with the selected fingerprint in the database 610. In block 602, the detection circuitry determines in this manner whether the sensor device is attached around the chest or the abdominal area. If the sensor device is detected to be around the abdominal area, the process proceeds to block 604 where the detection circuitry enables the first measurement circuitry for the first measurement mode (body composition measurements). If the sensor device is detected to be around the chest, the process proceeds to block 606 where the detection circuitry disables (or keeps disabled) the first measurement circuitry. The process may return to block 602 from block 606 to loop until the sensor device is detected around the abdominal area or alternatively end, as illustrated in Figure 6.

The embodiment of Figure 6 may be combined with the embodiments where the training computer instructs the user to attach the sensor device around the abdominal area. The procedure of Figure 6 may be used to replace the user acknowledgment by the automated detection procedure. Block 600 may be executed upon outputting the user instructions to attach the sensor device around the abdominal area, and the reception of the body composition measurement data may occur upon executing block 604.

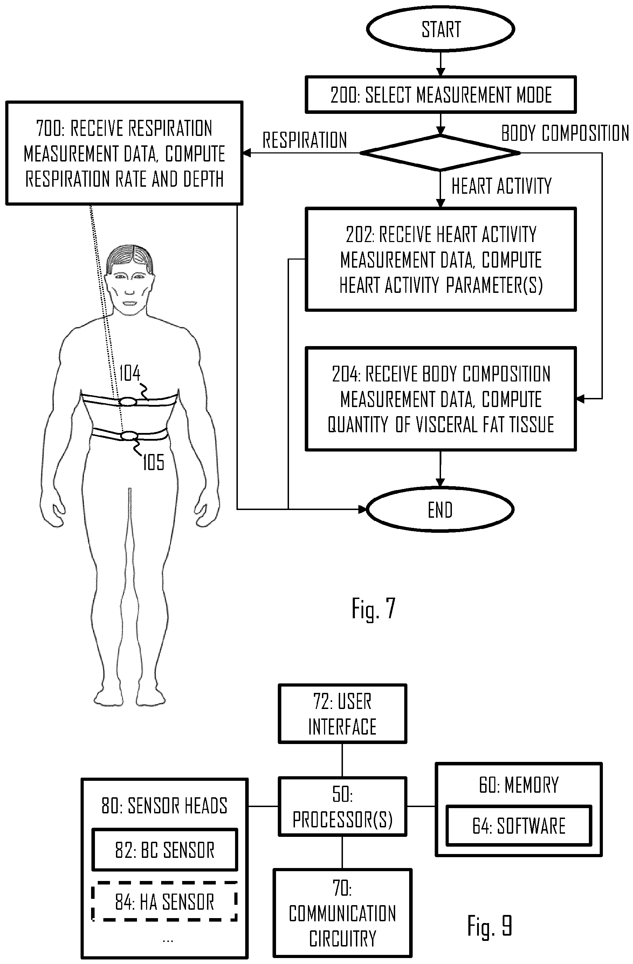

As described above, the same sensor device 104, 105 may be configured to carry out different measurements, depending on the attachment location. Figure 7 illustrates another embodiment where both sensor devices 104, 105 are used: the sensor device 104 attached around the chest area and the sensor device 105 attached around the abdominal area. In this embodiment, the two sensor devices may be used in cooperation to measure, for example, respiration of the user. For that purpose, each sensor device 104 and 105 or at least one of the sensor devices 104, 105 may comprise a motion sensor configured to measure the respiration of the user and the strap arranged to attach the sensor device around the torso of the user.

In this embodiment, the at least one processor supports a third measurement mode where the sensor device 104 is attached around the chest of the user while the other sensor device 105 is attached around the abdominal area of the user. The motion sensors of the sensor devices 104, 105 may measure and transmit respiration measurement data to the processor(s), and the processor(s) may compute respiration rate and respiration depth on the basis of the received respiration measurement data and output the computed respiration rate and respiration depth via the interface. As with the embodiments described above, the processor(s) may be comprised in the sensor device 104 or in the training computer. Figure 7 illustrates an embodiment of such a method where the processor(s) support the three measurement modes. In Figure 7, the operations denoted by the same reference numbers as in the other Figures represent the same or substantially similar operations. Accordingly, the processor(s) may support the body composition measurement mode and the heart activity measurement mode in the above-described manner. Referring to Figure 7, the processor(s) may additionally support the third measurement mode: a respiration measurement mode where the (at least) two sensor devices are used to measure the respiration. Upon triggering the respiration measurement mode, the process proceeds to block 700 where the processor(s) receive respiration measurement data from the two sensor devices 104, 105, and compute the respiration rate and the respiration depth. The actual measurement of the respiration rate and depth by using motion sensors is readily known in the art, and detailed description of that is omitted. It suffices to say that the respiration rate may be measured by measuring oscillation of the motion while the respiration depth may be measured by measuring the amplitude of the motion of each motion sensor in the direction perpendicular to the surface of the torso.

The placement of the sensor devices may be instructed to the user analogously to the embodiments described above for the body composition measurement mode. The only difference may be that now the user is instructed to place the two sensor devices around the torso: one around the chest and the other around the abdominal area.

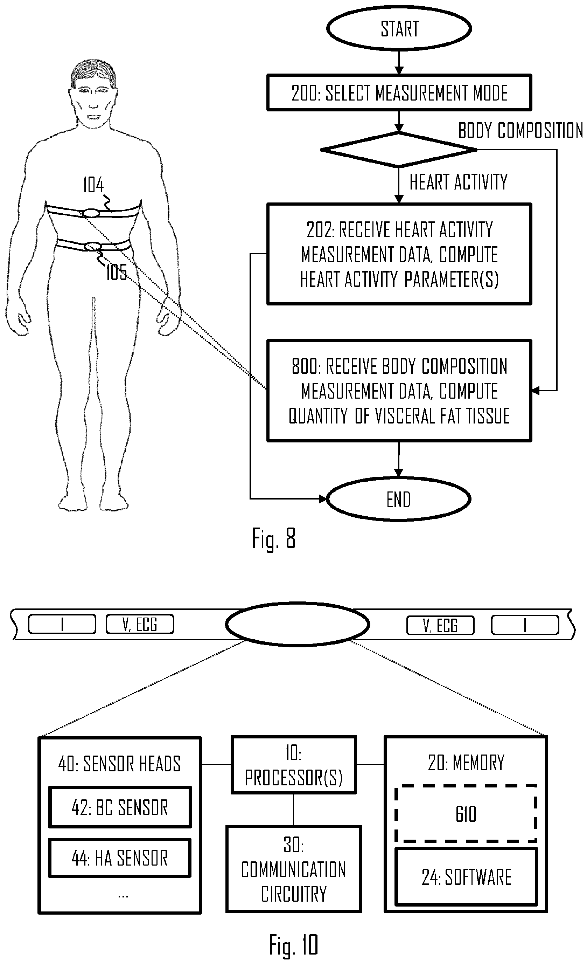

Figure 8 illustrates an embodiment using the same configuration of multiple sensor devices 104, 105 but, instead of using them in the third measurement mode measuring the respiration, they are used in the body composition measurement mode to improve the accuracy of the body composition measurements. In other words, at least three different measurement configurations may be used in the body composition measurements (block 800) according to this embodiment. Two measurement configurations are those described above: one using the sensor device 105 around the abdominal area and one using the body composition measurement circuitry of the wrist device. A further measurement configuration is gained by using another sensor device 104 attached around the chest area of the user. The sensor devices 104, 105 may have identical or similar straps to attach them around the torso. Alternatively, or additionally to the chest sensor device 104, the additional sensor device may be attached around a limb such as arm or leg. A suitable attachment position could be thigh or upper arm, for example, where the amount of muscular and fat tissue may vary between different users. The sensor may be attached to the limb by using a strap, a sleeve, or another equivalent attachment item.

Figure 9 an embodiment of an apparatus for the training computer. Referring to Figure 9, the apparatus may comprise at least one of the above-described at least one processor 50 configured to carry out the process of Figure 2 of any one of the above-described embodiments thereof. The apparatus may further comprise a user interface 72 comprising a display device, an input device and optionally a loudspeaker. The display device may comprise a touch-sensitive display functions as both the display and the input device. The input device may be configured to receive the above-described user inputs from the user of the training computer. The apparatus may further comprise at least some of the above-described sensors 080 for measuring the user. The sensor(s) 80 may comprise electronics required for measuring the desired characteristics. For example, the body composition sensor 82 may comprise electrodes for measuring the bioimpedance and for processing the bioimpedance measurement data into body composition components: a quantity of fat tissue, a quantity of bone tissue, a quantity of muscle tissue. The heart activity sensor 84 may have various electronics, depending on whether it comprises the ECG sensor and/or the PPG senso. For example, the ECG sensor head may require electronics and related ECG signal processing components, while a PPG sensor head requires at least one optical emitter, at least one photo detector, and related signal processing components.

The apparatus may further comprise a communication circuitry 70 that may comprise a radio modem, radio frequency components, and signal processing components implementing wireless communications according to at least one wireless communication protocol. An example of such a protocol is any commercial implementation of IEEE 802.15 standard. Bluetooth^{®} is an example of such a protocol.

The apparatus may further comprise the at least one memory 60 configured to store at least one computer program product 64 that configures the at least one processor 50 to carry out the above-described computer-implemented processes for measuring and computing the body composition, when the at least one computer program product is read and executed by the at least one processor.

Figure 10 illustrates an embodiment of an apparatus for the sensor device 104, 105. As described above, the apparatus may comprise the measurement circuitries realizing the sensor heads 42 for a body composition sensor 42 and for a heart activity sensor 44. As described above, each sensor may comprise a measurement circuitry comprise respective electronics and signal processing functions. For example, the body composition sensor 42 may comprise bioimpedance electrodes such as the current feed electrode(s) and voltage measurement electrode(s) in the strap, as illustrated in Figure 10. And as described above, the heart activity sensor 44 may comprise an electrode or electrodes in the strap for measuring the ECG and signal processing circuitries for processing the measured ECG signals. At least some of the electrodes may be coupled to both the body composition sensor 42 and the heart activity sensor 44.

The apparatus may comprise at least one of the above-described at least one processor. The processor(s) 10 of the sensor device may be configured, in some embodiments, to carry out the procedure of Figure 2 or any one of the embodiments thereof. In other embodiment, the processor(s) 10 of the sensor device is configured to control enablement/disablement of the measurement circuitries of the sensor device (see Figure 4 and associated description). In an embodiment, the processor(s) is configured to implement the detection circuitry described above. In the latter embodiments, the processor(s) may process the measurement signals received from the measurement circuitries and transmit the measurement data to the training computer for further processing, e.g. for the computation of the body composition and associated quantities of various tissues.

The apparatus may further comprise a communication circuitry 30 that may comprise a radio modem, radio frequency components, and signal processing components implementing wireless communications according to at least one wireless communication protocol. An example of such a protocol is any commercial implementation of IEEE 802.15 standard. Bluetooth^{®} is an example of such a protocol.

The apparatus may further comprise the at least one memory 20 configured to store at least one computer program product 24 that configures the at least one processor 10 to carry out the above-described computer-implemented processes for the sensor device 104, 105, when the at least one computer program product is read and executed by the at least one processor 10. The at least one memory may further store the database 610.

As used in this application, the term 'processor' refers to all of the following: (a) hardware-only circuit implementations, such as implementations in only analogue and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'processor' applies to all uses of this term in this application. As a further example, as used in this application, the term 'processor' would also cover an implementation of merely a processor (or multiple processors) or a portion of a processor and its (or their) accompanying software and/or firmware.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), graphics processing units (GPUs), processors, controllers, micro-controllers, microprocessors, other electronic units designed to perform the functions described herein, or a combination thereof. For firmware or software, the implementation can be carried out through modules of at least one chipset (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

It will be obvious to a person skilled in the art that, as the technology advances, the inventive concept can be implemented in various ways. The invention and its embodiments are not limited to the examples described above but may vary within the scope of the claims.

## Claims

1. A wearable body composition measurement system, comprising:
a sensor device (104) comprising a first measurement circuitry configured to measure body composition, a second measurement circuitry configured to measure heart activity, and a strap arranged to attach the sensor device around a torso of a user;
at least one processor (50) coupled with the sensor device (104); and
at least one memory (60) storing a computer program code configured to cause the at least one processor to perform operations comprising:
in a first measurement mode where the sensor device is attached around an abdominal area of the user (100), receiving body composition measurement data measured by the first measurement circuitry, computing a quantity of fat tissue in the abdominal area on the basis of the received body composition measurement data, and outputting the quantity of fat tissue via an interface; and
in a second measurement mode where the sensor device (104) is attached around a chest of the user, receiving heart activity measurement data from the second measurement circuitry, computing at least one heart activity parameter on the basis of the received heart activity measurement data, and outputting the at least one heart activity parameter via the interface;
further comprising a training computer (102) housing the at least one processor and the at least one memory and further comprising: a wireless communication circuitry configured to couple the at least one processor with the sensor device that is external to the training computer; a third measurement circuitry configured to measure a body composition, comprising a set of electrodes (300,302) arranged to contact with limbs of the user, wherein the at least one processor (50) is configured in the first measurement mode to control the third measurement circuitry to measure the body composition via the set of electrodes (300,302) and to generate further body composition measurement data, to compute a further quantity of fat tissue on the basis of said further body composition measurement data, and to output the further quantity of fat tissue via the interface, and the at least one processor (50) is configured to combine the body composition measurement data with the further body composition measurement data.

2. The wearable body composition measurement system of claim 1, wherein the training computer (102) is a wrist device.

3. The wearable body composition measurement system of any preceding claim, wherein the at least one processor (50) is configured to:
enable the first measurement circuitry and disable the second measurement circuitry in the first measurement mode; and
enable the second measurement circuitry and disable the first measurement circuitry in the second measurement mode.

4. The wearable body composition measurement system of any preceding claim, wherein the at least one processor (50) is configured to, upon triggering the first measurement mode, to output an instruction to the user to place the sensor device (104) around the abdominal area and, in response to a user input indicating that the sensor device (104) has been placed around the abdominal area, configure the first measurement circuitry to start measuring the body composition measurement data.

5. The wearable body composition measurement system of any preceding claim 1 to 4, wherein:
the first measurement circuitry is enabled in the first measurement mode and in the second measurement mode, and
the at least one processor (50) is configured to, upon triggering the first measurement mode, to output an instruction to the user to place the sensor device (104) around the abdominal area and, in response to a user input indicating that the sensor device has been placed around the abdominal area, start collection of the body composition measurement data for said computing the quantity of fat tissue in the abdominal area, wherein the at least one processor (50) is configured to exclude from said computing at least some of body composition measurement data received before the user input.

6. The wearable body composition measurement system of any preceding claim 1 to 5, wherein:
the at least one memory (60) stores a database comprising first information on a measurement signal fingerprint of a situation where the sensor device (104) is attached around the chest and second information on a measurement signal fingerprint of a situation where the chest first measurement circuitry is attached around the abdominal area, and
the sensor device comprises a detection circuitry configured to detect, by using the database and the first measurement circuitry, the second measurement circuitry, or another measurement circuitry of the sensor device, whether the sensor device is attached around the chest or the abdominal area and, if the sensor device is detected to be around the abdominal area, enable the first measurement circuitry for the first measurement mode, while if the sensor device is detected to be around the chest, disable the first measurement circuitry.

7. The wearable body composition measurement system of any preceding claim, wherein the first measurement circuitry comprises at least one of the following sensors configured to provide the body composition measurement data: a bioimpedance sensor, a radio frequency scanner, an ultrasound scanner, and an optical scanner.

8. The wearable body composition measurement system of any preceding claim, comprising a further sensor device comprising a further measurement circuitry configured to measure said body composition,
wherein the at least one processor is configured, in the first measurement mode where the further sensor device is attached around the chest of the user or around a limb of the user, to receive further body composition measurement data measured by the further measurement circuitry, to compute a further quantity of fat tissue in the attachment location of the further sensor device on the basis of the received further body composition measurement data, and to output the further quantity of fat tissue via the interface.

9. The wearable body composition measurement system of any preceding claim 1 to 8, comprising a further sensor device comprising a motion sensor configured to measure respiration of the user and a strap arranged to attach the sensor device around the torso of the user,
wherein the sensor device also comprises a motion sensor configured to measure the respiration of the user, and
wherein the at least one processor is configured, in a third measurement mode where one of the sensor device and the further sensor device is attached around the chest of the user while the other one of the sensor device and the further sensor device is attached around the abdominal area of the user, receive respiration measurement data from both said sensor device (104) and the further sensor device (102), to compute respiration rate and respiration depth on the basis of the received respiration measurement data, and to output the computed respiration rate and respiration depth via the interface.

10. A computer-implemented method comprising:
connecting with a sensor device (104) comprising a first measurement circuitry configured to measure body composition, a second measurement circuitry configured to measure heart activity, and a strap arranged to attach the sensor device (104) around a torso of a user;
in a first measurement mode where the sensor device (104) is attached around an abdominal area of the user, receiving body composition measurement data measured by the first measurement circuitry, computing a quantity of fat tissue in the abdominal area on the basis of the received body composition measurement data, and outputting the quantity of fat tissue via an interface; and
in a second measurement mode where the sensor device (104) is attached around a chest of the user, receiving heart activity measurement data from the second measurement circuitry, computing at least one heart activity parameter on the basis of the received heart activity measurement data, and outputting the at least one heart activity parameter via the interface;
communicating with an external training computer (102) comprising a third measurement circuitry configured to measure a body composition; and
measuring the body composition via the training computer (102) to: generate further body composition measurement data; the quantity of fat tissue on the basis of said further body composition measurement data; and to output the further quantity of fat tissue via the interface, and combine the body composition measurement data with the further body composition measurement data.

11. A computer program product embodiment on a computer-readable distribution medium storing computer program instructions that, when read and executed by a computer, cause the computer to carry out a computer process comprising:
connecting with a sensor device (104) comprising a first measurement circuitry configured to measure body composition, a second measurement circuitry configured to measure heart activity, and a strap arranged to attach the sensor device around a torso of a user;
in a first measurement mode where the sensor device (104) is attached around an abdominal area of the user, receiving body composition measurement data measured by the first measurement circuitry, computing a quantity of fat tissue in the abdominal area on the basis of the received body composition measurement data, and outputting the quantity of fat tissue via an interface; and
in a second measurement mode where the sensor device (104) is attached around a chest of the user, receiving heart activity measurement data from the second measurement circuitry, computing at least one heart activity parameter on the basis of the received heart activity measurement data, and outputting the at least one heart activity parameter via the interface;
communicating with an external training computer (102) comprising a third measurement circuitry configured to measure a body composition; and
measuring the body composition via the training computer (102) to: generate further body composition measurement data; the quantity of fat tissue on the basis of said further body composition measurement data; and to output the further quantity of fat tissue via the interface, and combine the body composition measurement data with the further body composition measurement data.
